# EUROPEAN PATENT APPLICATION

(11) **EP 4 492 037 A1**
(43) Date of publication of application: **15.01.2025**
(21) Application number: 24186808.2
(22) Date of filing: 05.07.2024
(51) Int. Cl.: G01N 15/14

(54) **METHOD FOR DETECTING PLATELET AGGREGATION**

(30) Priority: 10.07.2023 JP 2023113305; 10.07.2023 JP 2023113309; 10.07.2023 JP 2023113311
(71) Applicant: SYSMEX CORPORATION, Kobe-shi Hyogo 651-0073 (JP)
(72) Inventor: MATSUBA, Hiroaki, Kobe-shi, Hyogo, 651-0073 (JP)
(74) Representative: Hoffmann Eitle

(57) **Abstract**

Disclosed is a method for detecting platelet aggregation, the method including:
acquiring first scattered light information and second scattered light information generated by irradiating a measurement sample that contains particles with a light of a first wavelength and a light of a second wavelength; and
acquiring information that indicates presence or absence of platelet aggregation, with reference to the first scattered light information and the second scattered light information,
the first wavelength being 315 nm or longer and 600 nm or shorter, and the second wavelength being 610 nm or longer and 750 nm or shorter.

## Description

### FIELD OF THE INVENTION

The present invention relates to a method for detecting platelet aggregation.

### BACKGROUND

Blood includes various blood cell components such as red blood cell, white blood cell, and platelet. Among them, platelet is a non-nucleated cell having a diameter of 2 to 3 µm, whose count in normal blood is 150,000/µL to 350,000/µL. The platelet count has, however, been known to decrease in blood of patients with idiopathic thrombocytopenic purpura, acute leukemia and so forth. The platelet count in blood fallen below 10,000/µL is a widely-accepted criteria for need of blood transfusion. It is, therefore, important to exactly classify and count the platelet in clinical environment.

Platelet would aggregate in a blood collection tube, due to contamination with tissue fluid during blood collection, insufficient mixing, or action of EDTA. Platelet aggregation, whose size is different from that of a single platelet, is not counted as platelet, when analyzed with an automated hematology analyzer. Blood with the platelet aggregation occurred therein will, therefore, give an apparently decreased platelet count. This event, called pseudothrombocytopenia, can lead to misdiagnosis. A case suspected of pseudo thrombocytopenia needs blood collection and blood cell analysis once again.

U.S. Patent Application Publication No. 2020/0132589 describes a method for measuring platelet aggregation. In this prior document, red blood cell in a blood specimen is lysed with a hemolysis reagent, and the remaining blood cells are stained with a fluorescent dye to be analyzed with an automated hematology analyzer. The platelet aggregation is identified and counted on a scattergram created with reference to scattered light intensity and fluorescence intensity.

### SUMMARY OF THE INVENTION

It is therefore an object of the present invention to provide a novel means that enables detection of platelet aggregation.

The present inventors have found that platelet aggregation is detectable with reference to a first scattered light information and a second scattered light information, which are obtainable by irradiating the blood cells in a specimen with light having a wavelength of 315 nm or longer and 600 nm or shorter, and light having a wavelength of 610 nm or longer and 750 nm or shorter, respectively. The inventors have thus completed the inventions according to Items [1] to [13] below.
[1] A method for detecting platelet aggregation, the method including:
   acquiring first scattered light information and second scattered light information generated by irradiating a measurement sample that contains particles with a light of a first wavelength and a light of a second wavelength; and
   acquiring information that indicates presence or absence of platelet aggregation, with reference to the first scattered light information and the second scattered light information,
   the first wavelength being 315 nm or longer and 600 nm or shorter, and the second wavelength being 610 nm or longer and 750 nm or shorter.
[2] The method according to [1], in which the first scattered light information is information related to side-scattered light generated from the particles upon irradiation of the measurement sample with the light of the first wavelength, and
   the second scattered light information is information related to forward-scattered light generated from the particles upon irradiation of the measurement sample with the light of the second wavelength.
[3] The method according to [1] or [2], in which the measurement sample is prepared by mixing whole blood and a dilution reagent.
[4] The method according to any one of [1] to [3], in which the measurement sample is free of hemolysis reagent and fluorescent dye.
[5] The method according to any one of [1] to [4], further including, before acquiring the first scattered light information and the second scattered light information, preparing the measurement sample by mixing whole blood and a dilution reagent,
   the preparing does not include hemolyzing red blood cell contained in the measurement sample, and does not include staining blood cells contained in the measurement sample with a fluorescent dye.
[6] The method according to any one of [2] to [5], in which the information related to the side-scattered light is side-scattered light intensity, and the information related to the forward-scattered light is forward-scattered light intensity,
   in the step of acquiring information that indicates presence or absence of platelet aggregation:
   the particles in the measurement sample are classified into a platelet-containing population and a non-platelet blood cell-containing population, with reference to the side-scattered light intensity and the forward-scattered light intensity; and
   information that indicates presence of the platelet aggregation is acquired, if the platelet-containing population contains any particle whose forward-scattered light intensity is equal to or above a threshold value.
[7] The method according to [6], further including classifying the non-platelet blood cell-containing population into a red blood cell-containing population and a white blood cell-containing population, with reference to the side-scattered light intensity and/or the forward-scattered light intensity.
[8] The method according to [6] or [7], in which in the step of acquiring information that indicates presence or absence of platelet aggregation, a scattergram is created with reference to the side-scattered light intensity and the forward-scattered light intensity, and the platelet-containing population is identified on the scattergram.
[9] The method according to any one of [2] to [5], in which the information related to the side-scattered light is side-scattered light intensity, and the information related to the forward-scattered light is forward-scattered light intensity,
   the acquiring information that indicates presence or absence of platelet aggregation further includes:
   classifying the particles in the measurement sample into a while blood cell-containing population and a non-white blood cell-containing population, with reference to the side-scattered light intensity and the forward-scattered light intensity; and
   classifying the non-white blood cell-containing population into a platelet-containing population and a red blood cell-containing population, with reference to the forward-scattered light intensity,
   information that indicates presence of the platelet aggregation is acquired, if the platelet-containing population contains any particle whose forward-scattered light intensity is equal to or above a threshold value.
[10] The method according to [9], in which in the step of acquiring information that indicates presence or absence of platelet aggregation, create a scattergram is created with reference to the side-scattered light intensity and the forward-scattered light intensity, and the platelet-containing population is identified on the scattergram.
[11] The method according to any one of [2] to [5], in which the information related to the side-scattered light is side-scattered light intensity, and the information related to the forward-scattered light is forward-scattered light intensity, and
   in the step of acquiring information that indicates presence or absence of platelet aggregation, a scattergram is created with reference to the forward-scattered light intensity and the side-scattered light intensity, and a particle population that appears in a predetermined area is detected on the scattergram as the platelet aggregation.
[12] The method according to [11], in which in the step of acquiring information that indicates presence or absence of platelet aggregation, a scattergram is created with reference to the forward-scattered light intensity and the side-scattered light intensity, and at least one selected from the group consisting of white blood cell, platelet, and red blood cell is classified on the scattergram.
[13] The method according to any one of [1] to [12], in which the first wavelength is 315 nm or longer and 490 nm or shorter.

The present invention can provide a novel method for detecting platelet aggregation.

### BRIEF DESCRIPTION OF THE DRAWINGS

Fig. 1 is a perspective view illustrating an analysis system suitable for the method for detecting platelet aggregation of this embodiment.
Fig. 2 is a drawing illustrating a fluid circuit in a measurement unit, which involves a specimen suction unit, a sample preparation unit, and a flow cytometer (FCM) detector.
Fig. 3 is a block diagram illustrating a structure of the measurement unit.
Fig. 4 is a schematic drawing illustrating an exemplary structure of an optical system of the FCM detector.
Fig. 5 is a schematic drawing illustrating various kinds of light emitted from a particle that passes through a flow cell under irradiation with light, received by the optical system of the FCM detector.
Fig. 6 is a block diagram illustrating a structure of an analysis unit.
Fig. 7 is a flowchart illustrating a flow of operations of the analysis system.
Fig. 8 is a flowchart illustrating procedures of a measurement process.
Fig. 9 is a drawing illustrating an exemplary reagent for blood cell analysis of this embodiment.
Fig. 10 is a flowchart illustrating procedures of the analysis process in a first embodiment.
Fig. 11 contains schematic drawings, where (A) is a scattergram with the first side-scattered light (SSC-1) intensity plotted on the abscissa, and with the second forward-scattered light (FSC-2) intensity plotted on the ordinate, exemplifying positions where a PLT-containing population and a non-PLT blood cell-containing population appear; and (B) is the schematic drawing exemplifying a position where platelet aggregation appears in the scattergram named (A). "PLTc?" stands for particle suspected of forming platelet aggregation.
Fig. 12 is a flowchart illustrating procedures of the analysis process in a second embodiment.
Fig. 13 contains schematic drawings, where (A) is a scattergram with the SSC-1 intensity plotted on the abscissa, and with the FSC-2 intensity plotted on the ordinate, exemplifying positions where a WBC-containing population and a non-WBC blood cell-containing population appear; and (B) is the schematic drawing exemplifying a position where platelet aggregation appears in the scattergram named (A).
Fig. 14 is a flowchart illustrating procedures of the analysis process in a third embodiment.
Fig. 15 is a schematic drawing of a scattergram with the SSC-1 intensity plotted on the abscissa, and with the FSC-2 intensity plotted on the ordinate, exemplifying positions where an RBC-containing population, the WBC-containing population, the PLT-containing population, and platelet aggregation appear.
Fig. 16 contains scattergrams of a platelet aggregation (PLTc)-negative specimen and a PLTc-positive specimen 1 of Example 1.
Fig. 17 contains scattergrams of PLTc-positive specimens 2 and 3 of Example 1.
Fig. 18 contains scattergrams of PLTc-positive specimens 4 and 5 of Example 1.

Fig. 19 contains scattergrams of a measurement sample that contains a fluorescent dye, and a measurement sample free of fluorescent dye, of Example 2.

### DETAILED DESCRIPTION OF THE PREFERRED EMBODIMENTS

### (Analysis System)

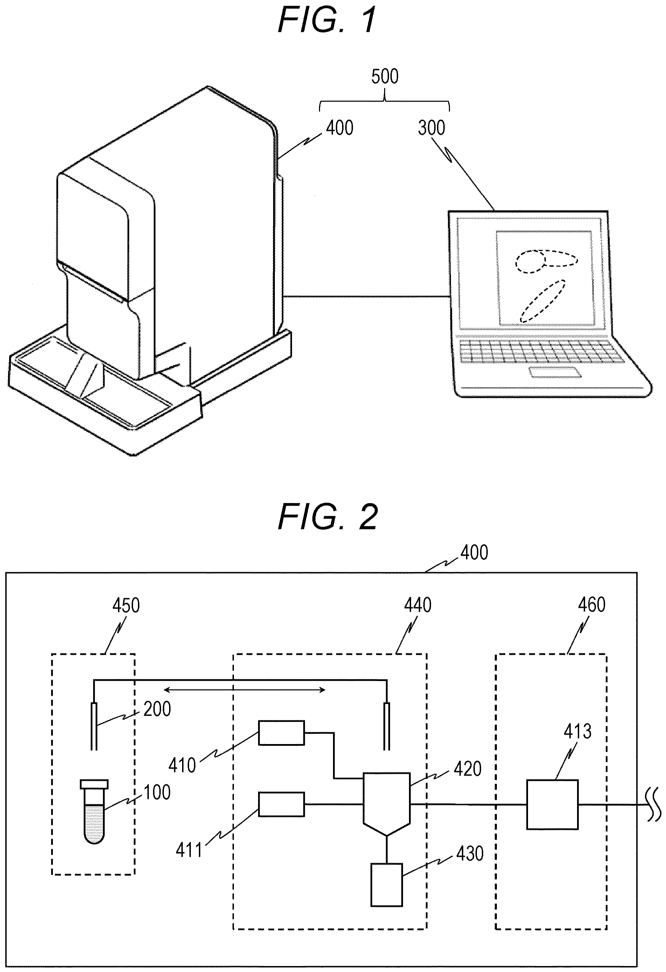

First, an analysis system suitable for a method for detecting platelet aggregation of this embodiment will be described, while referring to Fig. 1. An analysis system 500 has a measurement unit 400 as a measurement apparatus, and an analysis unit 300 as an analyzer. The analysis unit 300 is typically a personal computer having installed thereon software for analyzing specimen to be measured. The analysis unit 300 also takes part in operational control of the measurement unit 400. The analysis system 500 may alternatively be structured, with the analysis unit 300 incorporated in the measurement unit 400.

The measurement unit 400 is a unit for measuring the specimen, and contains a flow cytometer. In the measurement unit 400, the specimen and a reagent are mixed to prepare a measurement sample. The measurement sample is prepared with use of a dilution reagent as the reagent. The dilution reagent contains a buffering agent and a solvent. The specimen and the dilution reagent will be detailed later. In analysis of the particles in the specimen with this analysis system, particles in the measurement sample prepared from the specimen are analyzed. The "particles in the measurement sample" refers to elements contained in the measurement sample, which can be individually measured by an FCM detector 460 described later. The particles in the measurement sample are exemplified by cell contained in the specimen, red blood cell ghosts, platelet aggregation, lipid particle, fungi, and bacteria. The term "cell" encompasses various types of blood cell and platelet. The red blood cell ghost refers to a debris of hemolyzed red blood cell. The red blood cell ghost may be produced also due to hemolysis caused by other than the hemolysis reagent, that is, caused by physical impact on blood collection tube; or low pH or low osmotic pressure of the measurement sample.

The measurement sample is measured with an FCM detector 460 (see Fig. 2), described later, in the measurement unit 400. From the measurement, acquired is an optical signal related to scattered light emitted from the stained particles in the measurement sample. The method for detecting platelet aggregation of this embodiment, although without use of fluorescent dye, can also measure the particle stained with a fluorescent dye, and can acquire an optical signal emitted from the fluorescent dye on the particle. The "fluorescent dye on the particle" refers to a fluorescent dye, which is bound to the particle by staining. Mode of binding between the particle and the fluorescent dye is not particularly limited, as long as the particle and the fluorescent dye are integrally measurable by the flow cytometer. The acquired optical signals are subjected to A/D conversion. Digital data is thus acquired. The analysis unit 300 analyzes the digital data obtained by the measurement unit 400, to identify and/or classify the particles in the measurement sample.

A structure of a fluid system in the measurement unit 400 will be described, while referring to Fig. 2. The measurement unit 400 contains a sample preparation unit 440, a specimen suction unit 450, and the FCM detector 460. The sample preparation unit 440 contains a reagent container 410, a reagent container 411, a reaction chamber 420, and a waste liquid chamber 430. For example, the reagent container 410 contains the dilution reagent. A staining reagent may be contained in the reagent container 411, although the method for detecting platelet aggregation of this embodiment does not use the staining reagent. The staining reagent herein is a reagent that contains the fluorescent dye for staining the particles in the specimen, and a solvent therefor. The fluorescent dye per se has been known, and is commercially available. The reagent container 410 and the reagent container 411 are individually connected through a liquid feeding tube to the reaction chamber 420. In the sample preparation unit 440, the dilution reagent is injected through a liquid feeding tube into the reaction chamber 420. In a case where the particles are fluorescently stained, the staining reagent is injected through a liquid feeding tube into the reaction chamber 420, in the sample preparation unit 440. The specimen suction unit 450 contains a suction tube 200. The suction tube 200 sucks the specimen contained in the blood collection tube 100, and discharges the specimen into the reaction chamber 420. The reaction chamber 420 is connected through a liquid feeding tube, to the FCM detector 460.

The reaction chamber 420 is a container for preparing a measurement sample. The specimen and the dilution reagent are mixed in the reaction chamber 420, whereby the measurement sample is prepared. The measurement sample in the reaction chamber 420 is fed through the liquid feeding tube to the FCM detector 460, and measured. The FCM detector 460 acquires various optical signals emitted from each particle in the measurement sample. After completion of the measurement with the FCM detector 460, the measurement sample that remained in the reaction chamber 420 is discarded in the waste liquid chamber 430. The reaction chamber 420 is washed with an unillustrated washing mechanism, before preparation of the next measurement sample.

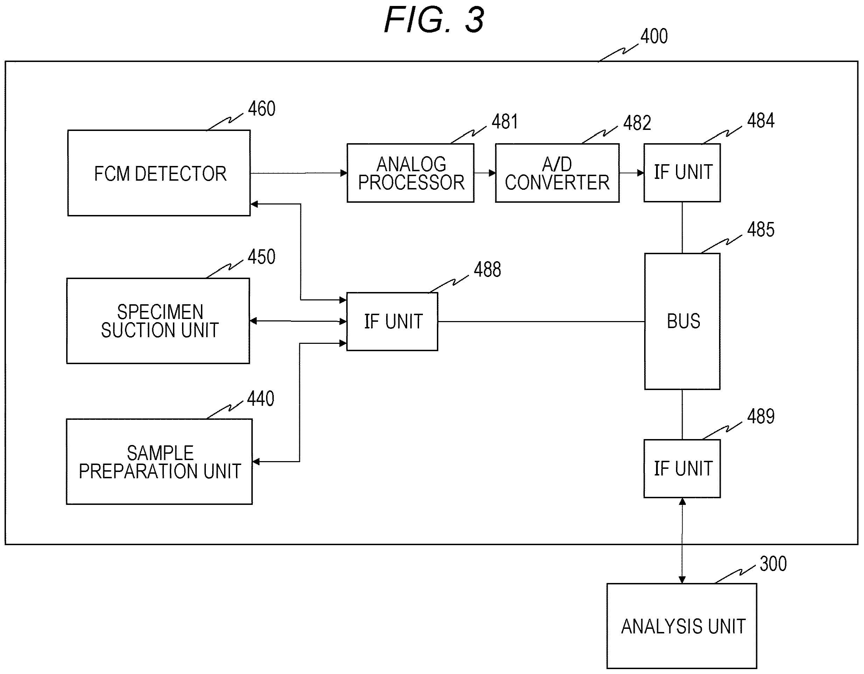

Electrical connection of the individual components in the measurement unit 400 will be described, while referring to Fig. 3. The sample preparation unit 440, the specimen suction unit 450, and the FCM detector 460 are connected to an interface (IF) unit 488. The FCM detector 460 is connected to an analog processor 481 and an A/D converter 482. Analog processor 481 processes an analog signal output from the FCM detector 460, and the A/D converter 482 converts the analog signal output from the analog processor 481 into a digital signal. The measurement unit 400 is connected through an IF unit 489 to the analysis unit 300. An IF unit 484, the IF unit 488, and the IF unit 489 are connected to a bus 485.

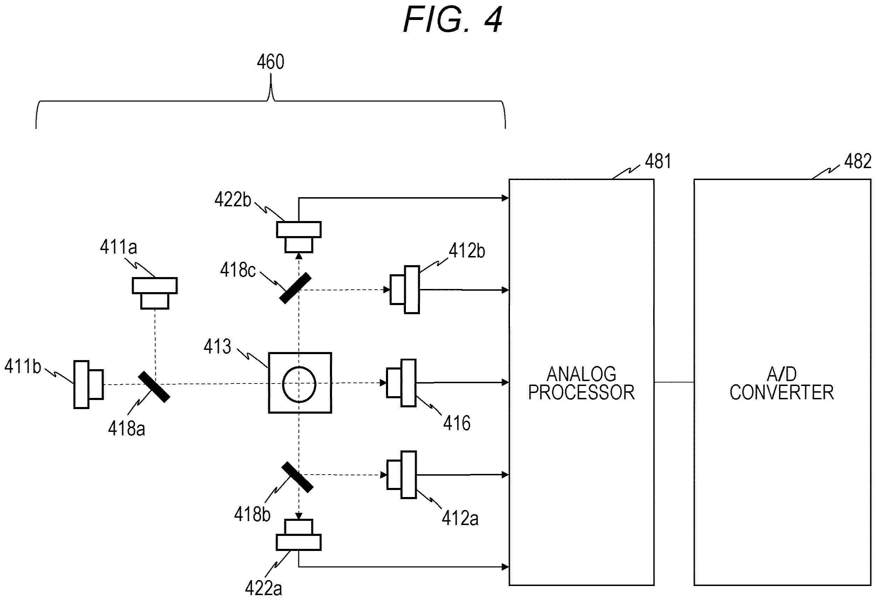

The FCM detector 460, the analog processor 481, and the A/D converter 482 will be described, while referring to Fig. 4. The FCM detector 460 has a first light source 411a, a second light source 411b, a flow cell 413, dichroic mirrors 418a, 418b, 418c, side-scattered light receivers 412a, 412b, a forward-scattered light receiver 416, and side-fluorescence receivers 422a, 422b. For the convenience of explanation of the optical system, Fig. 4 illustrates an exemplary structure capable of receiving the side-fluorescence that corresponds to the light emitted upon excitation of a fluorescent dye, although the method for detecting platelet aggregation of this embodiment does not use such fluorescent dye. The first light source 411a and the second light source 411b emit light of different wavelengths. For example, the first light source 411a emits light of a first wavelength, and the second light source 411b emits light of a second wavelength. The first wavelength is preferably a wavelength that hemoglobin can absorb. Such wavelength typically falls in the range from 315 nm or longer to 600 nm or shorter, preferably 400 nm or longer and 490 nm or shorter, more preferably 400 nm or longer and 450 nm or shorter, and yet more preferably 400 nm or longer and 410 nm or shorter. The light in the aforementioned wavelength range, when irradiated on red blood cell that contains hemoglobin, is absorbed by hemoglobin in the blood cell, and this tends to reduce parameters such as intensity (peak value), pulse width, and pulse area of scattered light from red blood cell, as compared with those from hemoglobin-free white blood cell. The second wavelength is different from the first wavelength, whose wavelength is preferably not absorbable by hemoglobin. Such wavelength typically falls in the range from 610 nm or longer to 750 nm or shorter, preferably 620 nm or longer and 700 nm or shorter, and more preferably 633 nm or longer and 643 nm or shorter. Use of lights in both wavelength ranges, absorbable by hemoglobin and not absorbable by hemoglobin, is considered to clearly discriminate red blood cell and white blood cell, thereby enabling more clear detection of platelet aggregation. A semiconductor laser light source, for example, is applicable to the first and second light sources.

The measurement sample prepared in the reaction chamber 420 is fed to the flow cell 413 of the FCM detector 460. In the illustration of Fig. 4, the measurement sample flows in a direction perpendicular to this page. With the measurement sample kept flowed through the flow cell 413, light emitted from the first light source 411a is reflected on the dichroic mirror 418a, and then irradiated on each particle in the measurement sample that flows through the flow cell 413. Light emitted from the second light source 411b transmits through the dichroic mirror 418a, and then irradiated on each particle in the measurement sample that flows through the flow cell 413.

In the example of Fig. 4, the forward-scattered light receiver 416 is arranged so as to receive forward-scattered light emitted from the particle upon being irradiated with the light from the second light source 411b. The light receiver 416 may alternatively be arranged so as to receive the forward-scattered light emitted from the particle upon being irradiated with the light from the first light source 411a. Still alternatively, an additional light receiver may be arranged so as to receive the forward-scattered light emitted from the particle upon being irradiated with the light from the first light source 411a. The forward-scattered light is typically a scattered light which may be received at a receiving angle of 0 to approximately 20 degrees. The forward-scattered light receiver 416 is typically a photodiode. The side-scattered light (first side-scattered light) that corresponds to the light irradiated by the first light source 411a is reflected on the dichroic mirror 418b, and then received by the side-scattered light receiver 412a. The side-scattered light (second side-scattered light) corresponded to the light irradiated by the second light source 411b is reflected on the dichroic mirror 418c, and then received by the side-scattered light receiver 412b. The side-scattered light is typically a scattered light which may be received at a receiving angle of approximately 20 degrees to approximately 90 degrees. The side-scattered light receivers 412a and 412b each are typically a photodiode.

The method for detecting platelet aggregation of this embodiment does not use any fluorescent dye. However, in a case where the particle is stained with a fluorescent dye which can be excited by the first wavelength, the side-fluorescence (first side-fluorescence) that corresponds to the light emitted from the excited fluorescent dye can transmit through the dichroic mirror 418b, and received by the side-fluorescence receiver 422a. Meanwhile in a case where the particle is stained with a fluorescent dye which can be excited by the second wavelength, the side-fluorescence (second side-fluorescence) that corresponds to the light emitted from the excited fluorescent dye can transmit through the dichroic mirror 418c, and received by the side-fluorescence receiver 422b. The side-fluorescence receivers 422a and 422b each is typically an avalanche photodiode.

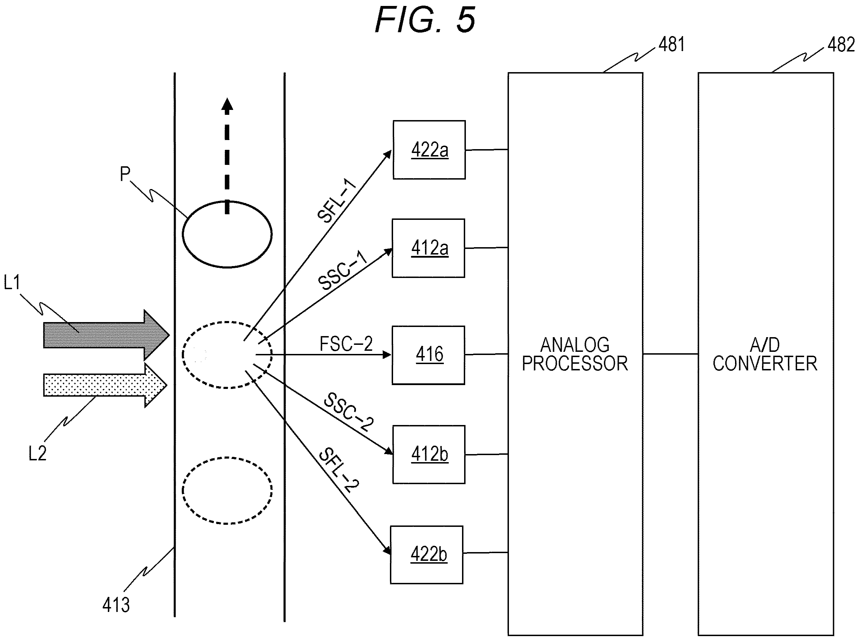

Relations of the various types of light emitted from a particle P that passes through the flow cell 413, with the optical system of the FCM detector 460 will be explained, while referring to Fig. 5. In Fig. 5, the light emitted from the first light source 411a is light L1 of the first wavelength, and the light emitted from the second light source 411b is light L2 of the second wavelength. Upon being irradiated with lights L1 and L2, the particle P that passes through the flow cell 413 emits forward-scattered lights that individually correspond to the light of the first wavelength and the light of the second wavelength, ahead of the direction of travel of light. Since the light receiver 416 herein receives the forward-scattered light that corresponds to the light of the second wavelength, Fig. 5 illustrates only the second forward-scattered light (FSC-2) that corresponds to the light of the second wavelength, while omitting the first forward-scattered light (FSC-1) that corresponds to the light of the first wavelength. Referring now to Fig. 5, there are generated the first side-scattered light (SSC-1) that corresponds to light of the first wavelength, and the second side-scattered light (SSC-2) that corresponds to the light of the second wavelength, emitted laterally with respect to the direction of travel of light. For the convenience of explanation of the optical system, Fig. 5 illustrates a state in which fluorescence occurred laterally with respect to the direction of travel of light, although the method for detecting platelet aggregation of this embodiment does not use any fluorescent dye. That is, with the particle P stained with the fluorescent dye that can be excited at the first wavelength, obtainable is the first side-fluorescence (SFL-1) excited with the light of the first wavelength, emitted laterally with respect to the direction of travel of light. Meanwhile, with the particle P stained with the fluorescent dye that can be excited at the second wavelength, obtainable is the second side-fluorescence (SFL-2) excited with the light of the second wavelength, emitted laterally with respect to the direction of travel of light.

As has been described, FSC-2, SSC-1, SFL-1, SSC-2, and SFL-2 are received by the light receivers 416, 412a, 422a, 412b, and 422b, respectively. Each light receiving element outputs a wavy electric signal (also referred to as an analog signal) that contains a pulse corresponded to the received light intensity. The analog signal corresponded to FSC-2 is also referred to as the "second forward-scattered light signal", the analog signal corresponded to the SSC-1 also as the "first side-scattered light signal", the analog signal corresponded to SFL-1 also as the "first fluorescence signal", the analog signal corresponded to SSC-2 also as the "second side-scattered light signal", and the analog signal corresponded to SFL-2 also as the "second fluorescence signal". In a case where FSC-1 is received, the analog signal corresponded to FSC-1 is also referred to as the "first forward-scattered light signal". One pulse of each analog signal corresponds to one particle (one cell, for example).

The analog signals corresponded to the various types of light are individually input to the analog processor 481, and subjected to processes such as noise removal and smoothing. The A/D converter 482 samples analog signals output from the analog processor 481, at a predetermined sampling rate (for example, 1024 points sampled at 10 nanosecond intervals, or 128 points sampled at 80 nanosecond intervals, or 64 points sampled at 160 nanosecond intervals). The A/D converter 482 digitizes the sampled analog signal, to produce waveform data. The A/D converter 482 samples and digitizes five types of analog signals that correspond to the individual cells that flow through the flow cell 413, to create waveform data including the second forward-scattered light signal, the first side-scattered light signal, the first fluorescence signal, the second side-scattered light signal, and the second fluorescence signal. In a case where FSC-1 is received, also waveform data of the first forward-scattered light signal is created. The A/D converter 482 also calculates a feature parameter that represents a morphological feature of each cell, from the waveform data of each signal. Such feature parameter is exemplified by peak value (height of pulse peak), pulse width, pulse area, transmittance, Stokes shift, ratio, temporal change, and values correlated thereto.

Optical information can be the aforementioned feature parameter. The optical information includes the first scattered light information and the second scattered light information. The first scattered light information relates to the side-scattered light generated from the particle, upon irradiation of the measurement sample with the light of the first wavelength. The second scattered light information relates to the forward-scattered light generated from the particle, upon irradiation of the measurement sample with the light of the second wavelength. Information on the side-scattered light is not particularly limited as long as it reflects internal information such as complexity of cellular structure, granule characteristic, nuclear structure, and degree of segmentation. Information on the forward-scattered light is not particularly limited as long as it reflects size of the particle. The first scattered light information is preferably peak value or pulse area of the first side-scattered light signal, and is more preferably the peak value of the first side-scattered light signal. The second scattered light information is preferably peak value or pulse area of the second forward-scattered light signal, and is more preferably the peak value of the second forward-scattered light signal. In this specification, the peak value of the first side-scattered light signal is also referred to as "first side-scattered light intensity", and the peak value of the second forward-scattered light signal is also referred to as "second forward-scattered light intensity".

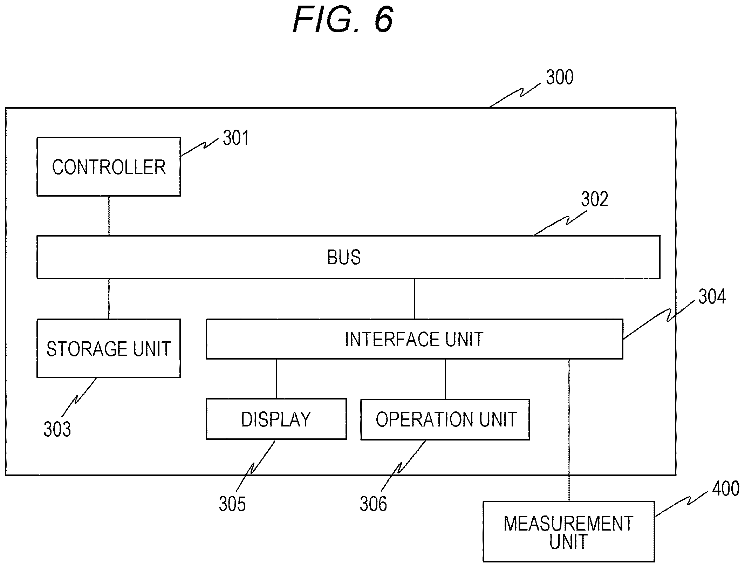

Referring now to Fig. 6, the analysis unit 300 is electrically connected through the interface unit 304 to the measurement unit 400. The interface unit 304 is typically an USB interface. The analysis unit 300 contains a controller 301, a bus 302, a storage unit 303, an interface unit 304, a display 305, and an operation unit 306. The analysis unit 300 is typically constituted by a personal computer (see the analysis unit 300 in Fig. 1), and executes a program product stored in the storage unit 303, to control the measurement unit 400 of the analysis system 500. The analysis unit 300 analyzes data that includes the optical information acquired by the measurement unit 400, and displays an analysis result on the display 305. The analysis unit 300 may also classify the cells with reference to the optical information.

The storage unit 303 typically stores a program product for controlling the measurement unit 400, and a program product for analyzing data acquired by the measurement unit 400. The display 305 typically displays result of analysis of data acquired by the measurement unit 400. The operation unit 306 has a keyboard, and a pointing device that contains a mouse or a touch panel.

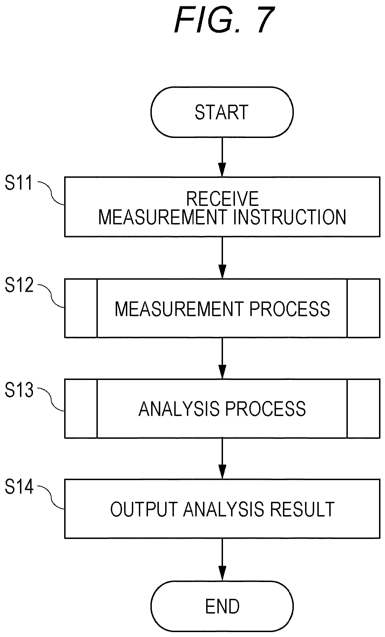

Exemplary operations of the analysis system 500 will now be described while referring to Fig. 7, without limiting the invention. In step S 11, the controller 301 of the analysis unit 300 receives a measurement start instruction from the user, through the operation unit 306. Upon receiving the measurement start instruction, the controller 301 transmits instruction data for instructing the measurement start to the measurement unit 400. The measurement unit 400 receives the instruction data, and executes a measurement process in step S12.

The method for detecting platelet aggregation of this embodiment typically encompasses a first embodiment, a second embodiment, and a third embodiment below. The "first embodiment" classifies the particles in the measurement sample into platelet and non-platelet blood cells with reference to the first scattered light information and the second scattered light information, and acquires information that indicates whether or not platelet contains platelet aggregation. The "second embodiment" classifies the particles in the measurement sample into white blood cell and blood cells other than white blood cell, with reference to the first scattered light information and the second scattered light information. The blood cells other than white blood cell are then classified into platelet and red blood cell, so as to acquire information that indicates whether or not platelet contains platelet aggregation. The "third embodiment" creates a scattergram with reference to the first scattered light information and the second scattered light information, and acquires information that indicates whether or not platelet contains platelet aggregation, on the scattergram.

### (Measurement Process)

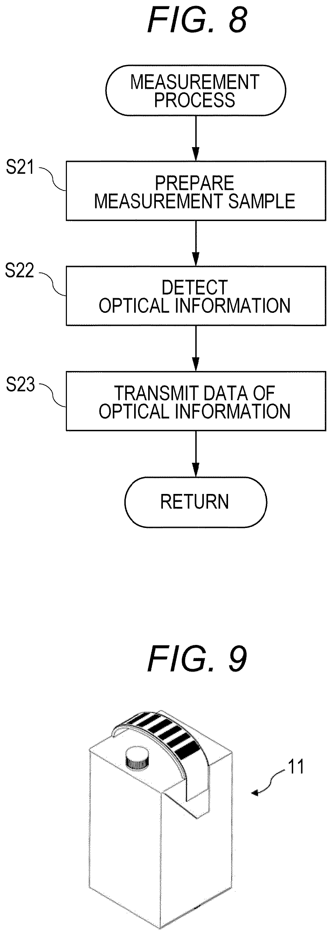

Exemplary measurement process in step S12 in Fig. 7 will be explained while referring to Fig. 8, without limiting the invention. The measurement process is common to all embodiments. In step S21, the controller 301 of the analysis unit 300 instructs the measurement unit 400 to prepare the measurement sample from the specimen. More specifically, the measurement unit 400 mixes the specimen and the dilution reagent in the reaction chamber 420, to prepare the measurement sample. In step S22, the controller 301 instructs the measurement unit 400 to feed the measurement sample to the FCM detector 460, and to implement optical measurement by flow cytometry. The measurement unit 400 thus detects optical information of each particle in the measurement sample, typically including waveform data of SSC-1 and FSC-2. In step S23, the controller 301 instructs the measurement unit 400 to transmit the optical information to the analysis unit 300. The measurement process thus finishes. The analysis unit 300 then implements the analysis process in step S13 in Fig. 7. Exemplary analysis processes of the individual embodiments will be described later.

### (Specimen)

The specimen is blood specimen. The blood specimen is exemplified by whole blood, and dilution of the whole blood. The whole blood is typically peripheral blood collected from the subject. The blood specimen may contain an anticoagulant. The anticoagulants is exemplified by ethylenediaminetetraacetate (EDTA), EDTA salts (EDTA 2K, EDTA 2Na, for example), sodium citrate, heparin, and warfarin. The dilution of whole blood is obtainable typically by diluting the whole blood with a suitable aqueous solvent, preferably with a dilution reagent described later. The aqueous solvent is exemplified by water, physiological saline, and aqueous solution of buffering agent.

The particles in the measurement sample are derived from blood components of the specimen. As referred herein, the "blood components" encompass the elements having been known to reside in blood, and abnormal cell. The elements having been known to reside in blood are typically blood cells usually contained in peripheral blood of healthy person. Such blood cells are exemplified by white blood cell, red blood cell, and platelet. The term "red blood cell" herein encompasses mature red blood cell and reticulocyte. The mature red blood cell is a terminally differentiated red blood cell having neither nucleus nor nucleic acid. The reticulocyte is a young red blood cell derived from erythroblastic cell in bone marrow, which is immediately after enucleated and released into peripheral blood. The "abnormal cell" refers to a cell that does not normally appear in blood.

The abnormal cell also encompasses non-cellular particle and microorganism. The non-cellular particle is exemplified by platelet aggregation, red blood cell ghost, and lipid particle. The microorganism is exemplified by bacteria and fungi. The platelet aggregation occurs in a blood collection tube, due to contamination with tissue fluid during blood collection, insufficient mixing, or action of EDTA.

### (Dilution Reagent)

The dilution reagent used for preparing the measurement sample will be described. The dilution reagent contains a buffering agent and a solvent. The dilution reagent preferably contains a solution of the buffering agent. Preferred example of the solvent includes water and physiological saline. Water is particularly preferred. The buffering agent preferably demonstrates a buffering function within a pH range from 6 or higher and 11 or lower. Such buffers can be selected typically from, carboxylate, phosphate, Good's buffer, taurine, and triethanolamine. The dilution reagent typically has a pH of 6 or higher and 11 or lower, which is preferably 7 or higher and 10 or lower, and more preferably 8 or higher and 9.5 or lower. With the dilution reagent whose pH is 6 or above, red blood cell will be less likely to hemolyze in the measurement sample. This successfully suppresses red blood cell ghost from occurring. With the dilution reagent whose pH is 11 or below, red blood cells and red blood cell ghost may be prevented from being non-specifically stained with the fluorescent dye. A preferred dilution reagent is an aqueous solution of the buffering agent, having a pH of 6 or higher and 11 or lower.

The dilution reagent may further contain, besides the buffering agent, components such as osmotic pressure compensator or preservative. The osmotic pressure compensator is a substance capable of maintaining osmotic pressure of the dilution reagent within an appropriate range. The osmotic pressure compensator is exemplified by alkali metal salt of organic acid such as propionic acid; saccharide such as glucose or mannose; alkali metal halide such as sodium chloride; and alkaline earth metal halide such as magnesium chloride. The osmotic pressure compensator may be used singly or in combination of two or more kinds thereof. The osmotic pressure compensator, when used, is added to the dilution reagent, so as to adjust the osmotic pressure of the dilution reagent to 150 mOsm/kg•H₂O or higher and 600 mOsm/kg•H₂O or lower, and more preferably to 200 mOsm/kg•H₂O or more and 300 mOsm/kg•H₂O or lower.

The preservative contained in the dilution reagent is exemplified by sodium 2-pyridylthio-1-oxide and β-phenethyl alcohol.

The method for detecting platelet aggregation of this embodiment may employ a commercially available dilution reagent. The commercially available dilution reagent is exemplified by CELLPACK (registered trademark) DFL (Sysmex Corporation).

The dilution reagent may also be provided as a reagent for blood cell analysis, used for the method for detecting platelet aggregation of this embodiment. The reagent for blood cell analysis of this embodiment may typically have a form such that the dilution reagent is filled in a container. Referring now to Fig. 9, reference sign 11 denotes a container, having the reagent for blood cell analysis of this embodiment filled therein. The container 11, when enclosed in a package box, may be typically accompanied by a package insert that describes composition of the reagent for blood cell analysis, directions for use, storage and so forth, and a cushion material for cushioning external impact, enclosed therein.

### (Preparation of Measurement Sample)

The ratio of mixing of the dilution reagent and the specimen may be appropriately determined, according to types of the specimen. The ratio of mixing of the dilution reagent and the specimen is preferably, for example, 1000 : 1 or larger on the volume basis. The ratio is more preferably 1000 : 5 or larger, and even more preferably 1000 : 10 or larger. As described above, the detection of platelet aggregation in this embodiment may employ the hemolysis reagent and the fluorescent dye, or does not have to employ the hemolysis reagent and the fluorescent dye. Hence, the measurement sample in the method for detecting platelet aggregation of this embodiment, does not contain the hemolysis reagent and the fluorescent dye. That is, the preparing the measurement sample, in the method for detecting platelet aggregation of this embodiment, includes neither lysing red blood cell, nor staining the blood cells with the fluorescent dye. The hemolysis reagent herein is a reagent for lysing red blood cell in a specimen, upon mixed with the specimen. The hemolysis reagent itself has been known, and typically contains a surfactant whose concentration is enough for lysing red blood cell.

### (Analysis Process in First Embodiment)

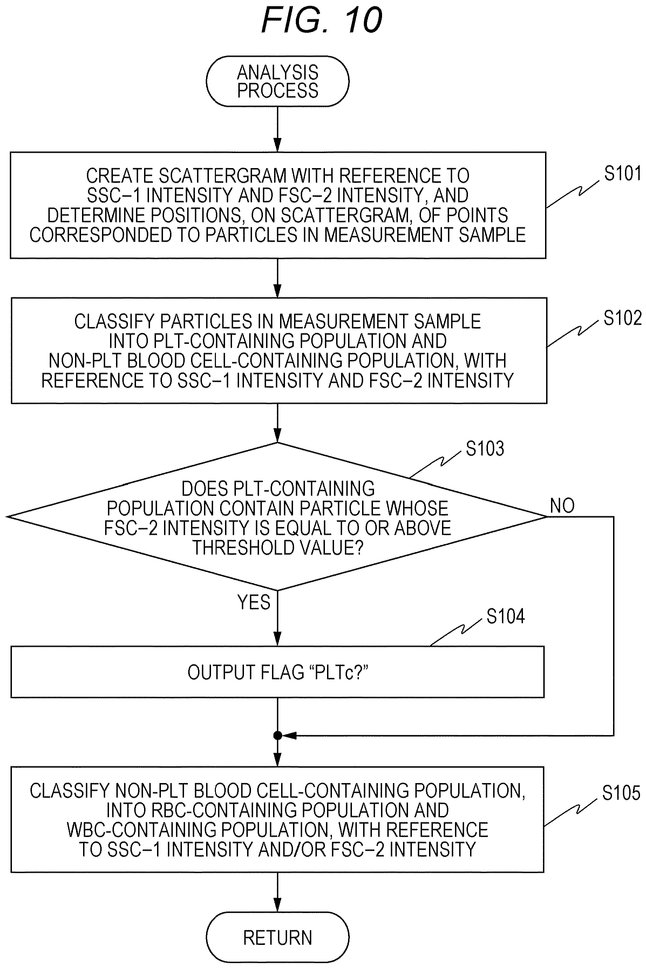

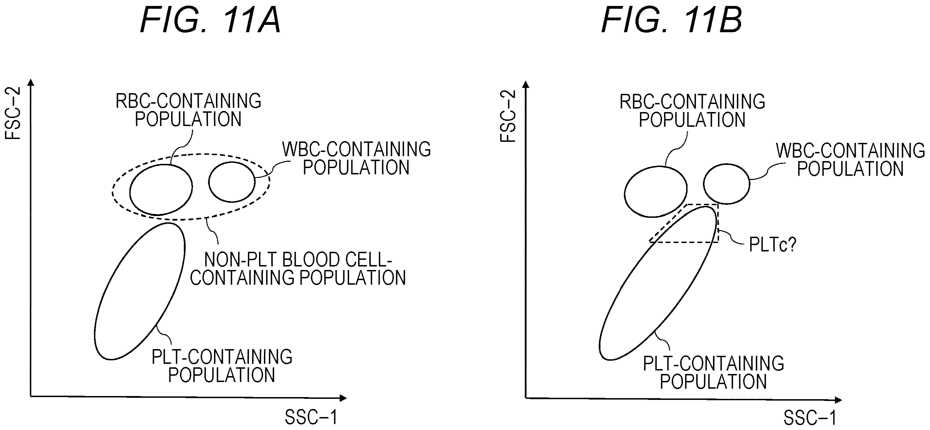

Exemplary analysis process in step S13 in Fig. 7 according to the first embodiment will be explained, while referring to Figs. 10 and 11, without limiting the invention. In this specification, platelet will be denoted as "PLT", red blood cell as "RBC", and white blood cell as "WBC". Referring now to Fig. 10, this analysis process first classifies the particles in the measurement sample into a PLT-containing population and a non-PLT blood cell-containing population. Since the method for detecting platelet aggregation of this embodiment does not use the hemolysis reagent, so that the measurement sample contains RBC. The measurement sample also contains WBC and PLT. That is, the non-PLT blood cells form a particle population that contains RBC and WBC. If the specimen contains platelet aggregation, then the PLT-containing population contains PLT and platelet aggregation. The PLT-containing population is identified with reference to the first scattered light information and the second scattered light information. This example employs the first side-scattered light intensity (also referred to as "SSC-1 intensity") as the first scattered light information, and the second forward-scattered light intensity (also referred to as "FSC-2 intensity") as the second scattered light information.

The PLT-containing population may be identified typically by analysis on scattergram. The scattergram is created typically with reference to the SSC-1 intensity and the FSC-2 intensity. Referring now to Fig. 10, the analysis unit 300 in step S 101 creates a scattergram having the SSC-1 intensity and the FSC-2 intensity plotted on two coordinate axes, with reference to the acquired optical information. Positions of points corresponded to the individual particles in the measurement sample are then determined, on the thus created scattergram. The abscissa and the ordinate of the scattergram may be expressed in either logarithmic or linear scale. The abscissa and the ordinate of the scattergram are preferably given in a logarithmic scale.

In step S102, the analysis unit 300 classifies the particles in the measurement sample into the PLT-containing population and the non-PLT blood cell-containing population, with reference to the SSC-1 intensity and the FSC-2 intensity. The non-PLT blood cell population demonstrates the FSC-2 intensity higher than that of the PLT-containing population. Now, the scattered light intensity of the particle populations on the scattergram is preferably compared, typically with use of statistical representative values of the scattered light intensity of the individual particle populations. The statistical representative value of the scattered light intensity of a certain particle population is a value that can be acquired from the scattered light intensity of all particles that constitute the particle population, and is exemplified by median value, mean value, and mode value. The median value is preferred.

Referring now to (A) in Fig. 11, the PLT-containing population appears on the scattergram in an area where the FSC-2 intensity is low. Meanwhile, the non-PLT blood cell-containing population appears in an area where the FSC-2 intensity is high. The non-PLT blood cell-containing population in (A) in Fig. 11 is a population constituted by particles that fall in an area surrounded by the broken line. More specifically, the non-PLT blood cell-containing population encompasses the RBC-containing population and the WBC-containing population. The PLT has the smallest size among the blood cells, and thus produces the forward-scattered light whose intensity is lower than that of RBC or WBC. The PLT population therefore appears on the scattergram, in the area where the FSC-2 intensity is lower than those of the RBC-containing population and the WBC-containing population. Thus the particles in the measurement sample may be classified into the PLT-containing population and the non-PLT blood cell-containing population.

Referring now to (B) in Fig. 11, illustrating a case where the specimen contains platelet aggregation, there are particles that distribute so as to extend from the PLT-containing population towards the WBC-containing population, found on the scattergram. In (B) in Fig. 11, particles that appear in an area surrounded by the broken line represent platelet aggregation. Platelet aggregation is an aggregate of a plurality of platelets, whose size is larger than that of normal platelet. The particles of platelet aggregation therefore demonstrate higher forward-scattered light intensity than platelet. Whether the PLT-containing population contains platelet aggregation or not can, therefore, be determined with reference to the FSC-2 intensity. Referring now to Fig. 10, the analysis unit 300 in step S103 determines whether or not the PLT-containing population contains any particle whose FSC-2 intensity is equal to or above a threshold value. More specifically, the FSC-2 intensity of all particles constituting the PLT-containing population is compared with a threshold value that corresponds to the FSC-2 intensity. The particles whose FSC-2 intensity is equal to or above the threshold value are suspected of forming the platelet aggregation. That is, the PLT-containing population is suspected of containing the platelet aggregation. If step S103 judged there are particles whose FSC-2 intensity is equal to or above the threshold value, the process advances to step S 104. Meanwhile, if step S103 judged there are no particle whose FSC-2 intensity is equal to or above the threshold value, the process advances to step S105.

In a further embodiment, particles whose FSC-2 intensity is equal to or above the threshold value may be counted. The cells are counted by the analysis unit 300. If step S103 judges the count of the particles, demonstrating the FSC-2 intensity equal to or above the threshold value, is equal to or above a predetermined value, the process advances to step S 104. Meanwhile, if the count of the particles, demonstrating the FSC-2 intensity equal to or above the threshold value, is below the predetermined value, the process advances to step S105. Now, the predetermined value is a value used for determining whether or not the particles, demonstrating the FSC-2 intensity equal to or above the threshold value, are platelet aggregation.

In step S104, the analysis unit 300 outputs information that indicates presence of platelet aggregation. For example, a flag "PLTc?" may be output on the display 305. No-display of the flag on the display 305 can be information that indicates absence of platelet aggregation. The process then advances to step S105. In step S105, the non-PLT blood cell-containing population is further analyzed. In step S105, the analysis unit 300 classifies the non-PLT blood cells into the RBC-containing population and the WBC-containing population, with reference to the SSC-1 intensity and/or the FSC-2 intensity. Preferably, the analysis unit 300 classifies the non-PLT blood cells into the RBC-containing population and the WBC-containing population, with reference to the SSC-1 intensity. Referring now to (A) and (B) in Fig. 11, on the scattergrams with reference to the SSC-1 intensity and the FSC-2 intensity, the RBC-containing population appears in areas where the SSC-1 intensity is lower than that of the WBC-containing population. It is widely known that RBC has a diameter of 7 to 8 µm, meanwhile WBC has a diameter of 7 to 21 µm. Hence, on the aforementioned scattergrams, the WBC population tends to appear in areas where the FSC-2 intensity is higher than that of the RBC-containing population. Thus the non-PLT blood cell population may be classified into the RBC-containing population and the WBC-containing population. How the RBC-containing population and the WBC-containing population can be classified with reference to the SSC-1 intensity will be explained below.

Hemoglobin contained in RBC has been known to absorb blue-violet light. RBC, when irradiated with the light of the first wavelength, which corresponds to blue-violet light, produces scattered light whose intensity is weaker than the scattered light expectedly produced from RBC irradiated with the light of the second wavelength, which corresponds to red light. Hence, the red light has been preferred for use in the prior hemanalysis. On the other hand, WBC demonstrates almost no difference in the intensity of the scattered light, either irradiated with the light of the first wavelength, or with the light of the second wavelength. The present inventors have arrived at an idea of discriminating WBC from RBC, making use of this finding. More specifically, the light of the first wavelength is irradiated to cause a difference in the first scattered light information, between WBC and RBC. For an exemplary case where the first scattered light information denotes the SSC-1 intensity, the SSC-1 intensity of RBC becomes lower due to absorption by hemoglobin. The SSC-1 intensity of the WBC, however, remains unaffected. The SSC-1 intensity of RBC therefore becomes lower than that of WBC. Hence the RBC-containing population and the WBC-containing population are discriminable with reference to the SSC-1 intensity. After completion of the classification of the non-PLT blood cells, the analysis unit 300 finishes the analysis process. The process then advances to step S14 in Fig. 7.

### (Analysis Process of Second Embodiment)

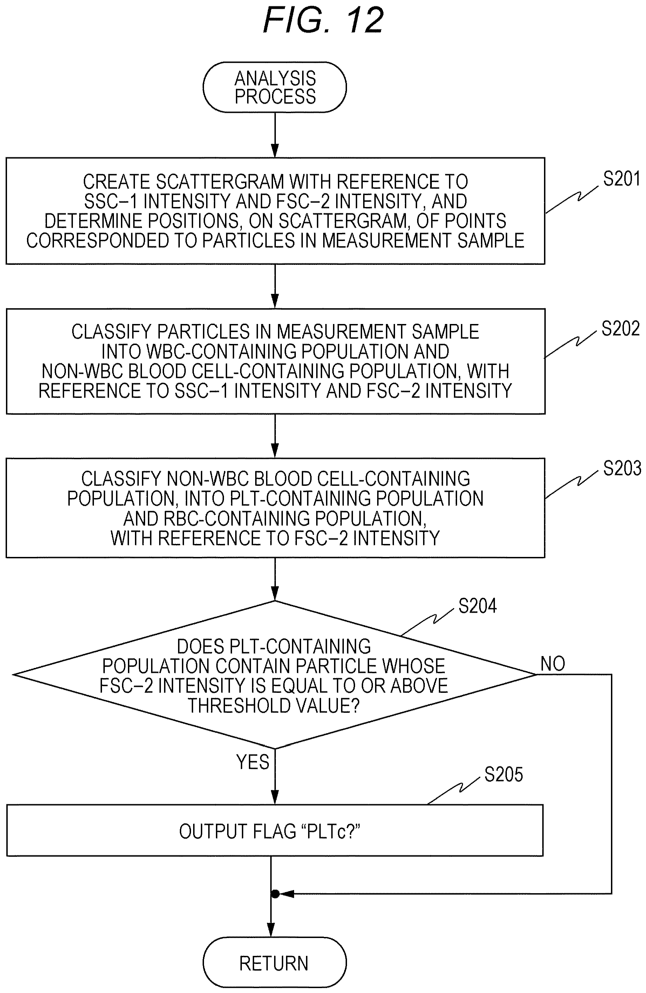

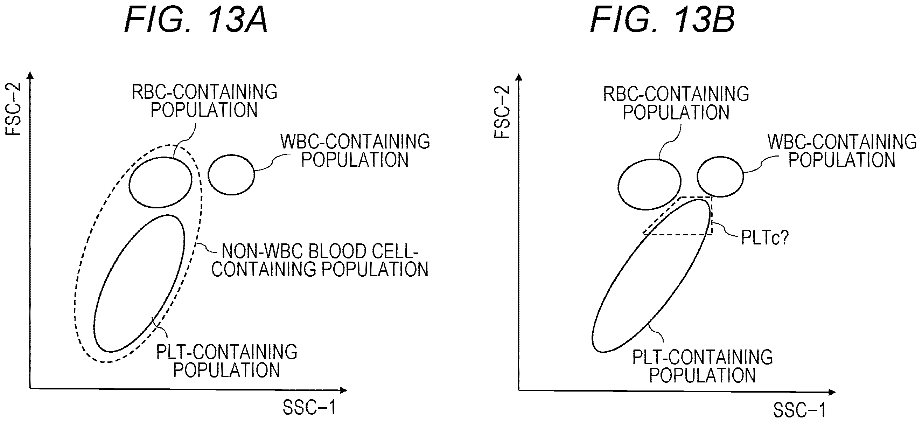

Exemplary analysis process in step S13 in Fig. 7 according to the second embodiment will be explained, while referring to Figs. 12 and 13, without limiting the invention. Referring now to Fig. 12, first, this analysis process classifies the particles in the measurement sample into the WBC-containing population and a non-WBC blood cell-containing population. As has been described previously, the method for detecting platelet aggregation of this embodiment does not use the hemolysis reagent, so that the measurement sample contains RBC. The measurement sample also contains WBC and PLT. That is, the non-WBC blood cells form a particle population that contains RBC and PLT. If the specimen contains platelet aggregation, then the non-WBC blood cell-containing population contains RBC, PLT, and platelet aggregation. The non-WBC blood cell-containing population is identified with reference to the first scattered light information and the second scattered light information. This example employs the SSC-1 intensity as the first scattered light information, and the FSC-2 intensity as the second scattered light information.

The non-WBC blood cell-containing population may be identified, typically by analysis on scattergrams. The scattergram is created typically with reference to the SSC-1 intensity and the FSC-2 intensity. Referring now to Fig. 12, the analysis unit 300 in step S201 creates a scattergram having the SSC-1 intensity and the FSC-2 intensity plotted on two coordinate axes, with reference to the acquired optical information. Positions of points corresponded to the individual particles in the measurement sample are then determined, on the thus created scattergram. Scaling of the abscissa and ordinate of the scattergram is as described previously.

In step S202, the analysis unit 300 classifies the particles in the measurement sample into the WBC-containing population and the non-WBC blood cell-containing population, with reference to the SSC-1 intensity and the FSC-2 intensity. The WBC-containing population demonstrates the SSC-1 intensity and the FSC-2 intensity higher than those of the non-WBC blood cell-containing population. Comparison of the scattered light intensity among the particle populations on the scattergrams is as described previously.

Referring now to (A) in Fig. 13, the WBC-containing population appears on the scattergram in an area where the SSC-1 intensity is high. Meanwhile, the non-WBC blood cell-containing population appears in an area where the SSC-1 intensity is low. The non-WBC blood cell-containing population in (A) in Fig. 13 is a population constituted by particles that fall in an area surrounded by the broken line. More specifically, the non-WBC blood cell-containing population encompasses the RBC-containing population and the PLT-containing population. As described previously, RBC contains hemoglobin, and the RBC-containing population therefore appears on the scattergram in the area where the SSC-1 intensity is lower than that of the WBC-containing population. Also since PLT has the smallest size among the blood cells, the PLT-containing population therefore appears on the scattergram, in the area where the FSC-2 intensity is lower than that of the WBC-containing population. As can be seen, the scattergram with reference to the SSC-1 intensity and the FSC-2 intensity can classify the particles in the measurement sample, into the WBC-containing population and the non-WBC blood cell-containing population. The analysis unit 300 then identifies the non-WBC blood cell-containing population.

Referring now to Fig. 12, the analysis unit 300 in step S203 classifies the non-WBC blood cell-containing population into the PLT-containing population and the RBC-containing population, with reference to the FSC-2 intensity. Since the PLT-containing population appears in the area where the FSC-2 intensity is lower than that of the RBC-containing population as described previously, so that the PLT-containing population and the RBC-containing population are discriminable with reference to the FSC-2 intensity.

Referring now to (B) in Fig. 13, illustrating a case where the specimen contains platelet aggregation, there are particles that distribute so as to extend from the PLT-containing population towards the WBC-containing population, found on the scattergram. In (B) in Fig. 13, particles that appear in an area surrounded by the broken line represent platelet aggregation. Since platelet aggregation is larger in size than normal platelet as described previously, so that whether or not platelet aggregation is contained in the PLT-containing population can be determined with reference to the FSC-2 intensity. Referring now to Fig. 12, the analysis unit 300 in step S204 determines whether or not the PLT-containing population contains any particle whose FSC-2 intensity is equal to or above a threshold value. This determination may be made by comparing the count of the particles, demonstrating the FSC-2 intensity equal to or above a threshold value, with a predetermined value. The determination is similar to that described for step S103. If step S204 judges there is any particle demonstrating the FSC-2 intensity equal to or above the threshold value (or, the count of the particles, demonstrating the FSC-2 intensity equal to or above the threshold value, is equal to or above a predetermined value), the process then advances to step S205.

Meanwhile, if step S204 judges there are no particle demonstrating the FSC-2 intensity equal to or above the threshold value (or, the count of the particles, demonstrating the FSC-2 intensity equal to or above the threshold value, is below the predetermined value), the analysis unit 300 finishes the analysis process. The process then advances to step S14 in Fig. 7. Step S205 is similar to that described for step S104. After outputting information that indicates presence of platelet aggregation, the analysis unit 300 finishes the analysis process. The process then advances to step S14 in Fig. 7.

### (Analysis Process in Third Embodiment)

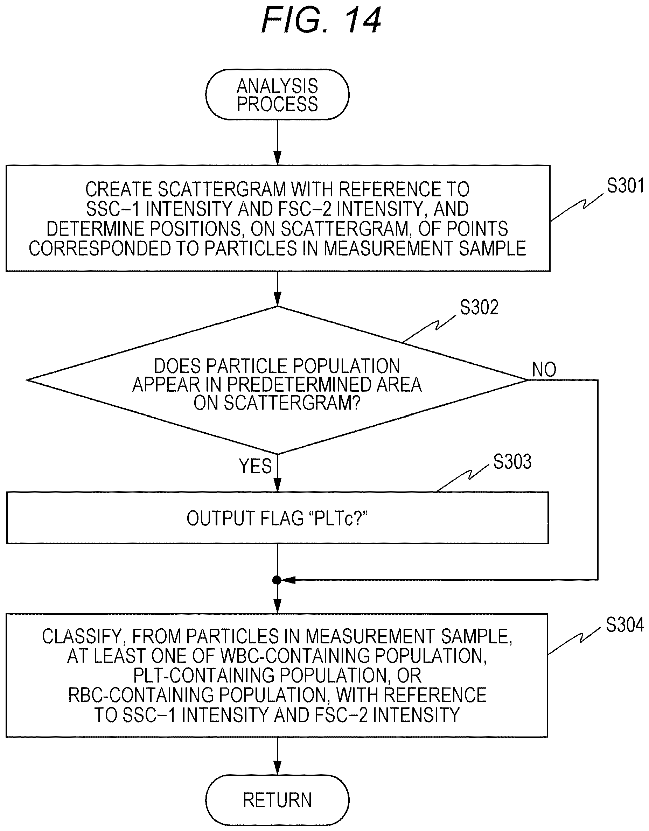

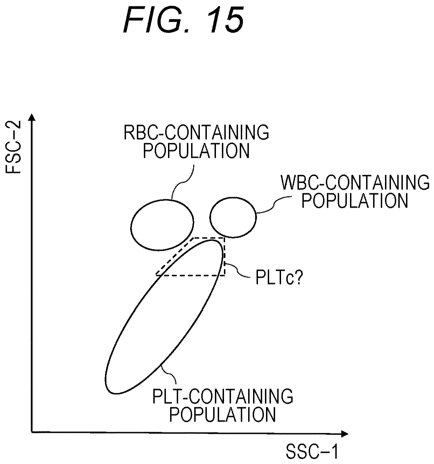

Exemplary analysis process in step S13 in Fig. 7 according to the third embodiment will be explained, while referring to Figs. 14 and 15, without limiting the invention. Referring now to Fig. 14, this analysis process detects platelet aggregation, on a scattergram created with reference to the SSC-1 intensity and the FSC-2 intensity. The analysis unit 300 in step S301 creates a scattergram having the SSC-1 intensity and the FSC-2 intensity plotted on two coordinate axes, with reference to the acquired optical information. Positions of points corresponded to the individual particles in the measurement sample are then determined, on the thus created scattergram. Scaling of the abscissa and ordinate of the scattergram is as described previously.

The analysis unit 300 in step S302 determines whether a population of particles appears in a predetermined area on the scattergram. For example, if at least one point that corresponds to each particle in the measurement sample is located in a predetermined area, such particle is determined to appear in the predetermined area. If there are a plurality of points that appear in the predetermined area, such predetermined area is judged to have a population of particles appeared therein. The process then advances to step S303. The count of the particles that appear in the predetermined area may only be two or more. The count of the particles is preferably equal to or above such predetermined value.

The predetermined area may be an area where the particles that form platelet aggregation are predicted to appear. Such area is exemplified in Fig. 15, as an area surrounded by the broken line. The particles that appear in this area represent the platelet aggregation. As has been described previously, the specimen, if containing platelet aggregation, will demonstrate the particles that distribute so as to extend from the PLT-containing population towards the WBC-containing population, on the scattergram. As illustrated in Fig. 15, the predetermined area may alternatively be determined from a positional relation among the WBC-containing population, the RBC-containing population, and a population that contains non-aggregated PLT. The predetermined area may alternatively be determined, typically by accumulating measurement data for a plurality of blood specimens known to contain platelet aggregation, and a plurality of normal blood specimens. These specimens are measured to create a scattergram, and distributions of the particles on the scattergram are compared, thereby accumulating the data of areas where the particles of platelet aggregation appear. From these data, then preliminarily determined is an area where platelet aggregation is predicted to appear, as the predetermined area.

If step S302 judged there is a particle population appeared in the predetermined area, the process then advances to step S303. Step S303 is similar to that described for step S104. Meanwhile, if there is no particle appeared in the predetermined area, or the count of the particle that appeared therein is one or less, step S302 judges there is no particle appeared in the predetermined area. The process then advances to step S304. Non-appearance of the particles in the predetermined area is preferably judged if the count of the particles that appear in the predetermined area is below the predetermined value.

In step S304, the analysis unit 300 classifies at least one of the WBC-containing population, the PLT-containing population, or the RBC-containing population, from the particles in the measurement sample, with reference to the SSC-1 intensity and the FSC-2 intensity. The WBC-containing population can be identified as a population whose SSC-1 intensity and/or FSC-2 intensity are highest on the scattergram. The PLT-containing population can be identified as a population whose FSC-2 intensity is lowest on the scattergram. The RBC-containing population can be identified as a population whose SSC-1 intensity is lower than that of the WBC-population, and whose FSC-2 intensity is higher than that of the PLT-containing population on the scattergram. Comparison of the scattered light intensity among the particle populations on the scattergrams is as described previously. After completion of the classification of the particles in the measurement sample, the analysis unit 300 finishes the analysis process. The process then advances to step S14 in Fig. 7.

Referring now to Fig. 7, upon completion of the aforementioned analysis process, the controller 301 in step S14 outputs analysis result on the display 305. The process thus finishes. Paragraphs above have described the analysis process which involves detection of platelet aggregation in the measurement sample, and analysis of at least one of white blood cell, platelet, or red blood cell, while referring to Figs. 10 to 15. The analysis process may, however, only be implemented in a part thereof, or may be implemented entirely.

If any particle suspected of platelet aggregation is identified, an analysis result screen that is output on the display 305 may contain information that indicates presence of platelet aggregation in the specimen. For example, a flag "PLTc?" may be output on the display 305. The screen may also contain the white blood cell count, the red blood cell count, and the platelet count. Besides these information, the screen may contain a scattergram created with reference to the optical information.

As has been described, the method for detecting platelet aggregation of this embodiment can provide a medical worker such as doctor or medical technician, with analysis result such as information on whether the specimen contains platelet aggregation or not. The method can also provide analysis results of white blood cell, red blood cell, and platelet. The medical worker can make decision from the acquired analysis results, on whether the specimen should further be inspected or not. All of the aforementioned preparation of the measurement sample and analysis of the specimen take place in vitro.

The present invention will be further detailed below, while referring to Examples, to which the present invention is however not limited.

### EXAMPLES

In the following Examples, the method for detecting platelet aggregation of this embodiment was implemented with use of the blood specimen, the dilution reagent, and an analyzer below. Details will be described later in [Method of Measurement].

### [Blood Specimen]

Whole blood (peripheral blood) was collected from a plurality of subjects, with use of an EDTA-2K blood collection tube, and stored at room temperature (25 ± 2°C). The whole blood was fractionated from the blood collection tube at the time of use, to be used as the blood specimen. Details of the blood cell components contained in the blood specimen and/or the subject will be described later in the individual Examples.

### [Dilution Reagent]

CELLPACK (registered trademark) DFL (Sysmex Corporation) was used as the dilution reagent.

### [Analyzer]

A prototype, modified from a flow cytometer XF-1600 (Sysmex Corporation) was used as the analyzer. The prototype was fabricated as follows. The original XF-1600 has been equipped with three semiconductor laser light sources that emit blue-violet (405 nm) light, blue light (488 nm) and red (638 nm) light, detectors for scattered light of the individual colors, and detectors for fluorescence excited by lights of the individual colors. The XF-1600 was then modified, by detaching the semiconductor laser light source that emits blue (488 nm) light, to an FCM system having two semiconductor laser light sources. The prototype has the hardware design illustrated in Figs. 1 to 3, and an FCM detector has a structure illustrated in Fig. 4.

### [Method of Measurement]

Preparation and measurement of the measurement sample were implemented according to a manual attached to XF-1600, except that staining reagent was not used, and that CELLPACK DFL was used. The measurement sample was prepared by mixing 1000 µL of CELLPACK DFL, and 5.0 µL of whole blood. The measurement sample was measured after incubation at 41°C for 30 seconds.

### Example 1: Analysis of Measurement Sample that Contains Platelet Aggregation

Measurement samples were prepared from blood specimens that contain platelet aggregation, and platelet aggregation in the measurement samples was detected with an analyzer.

### (1) Measurement Samples

Normal blood specimen (one specimen) and blood specimens that contain platelet aggregation (five specimens) were selected. Hereinafter, the normal blood specimen is also referred to as "PLTc-negative specimen". Meanwhile, the blood specimens that contain platelet aggregation will be referred to as "PLTc-positive specimen 1","PLTc-positive specimen 2", "PLTc-positive specimen 3","PLTc-positive specimen 4", and "PLTc-positive specimen 5", hereinafter. The PLTc-negative specimen was whole blood having been determined to be normal by analysis with an automated hematology analyzer XN-20 (Sysmex Corporation). The PLTc-positive specimens 1 to 5 were whole bloods having been determined to be platelet aggregation-positive, by visual observation under a microscope. From these blood specimens, the measurement samples were prepared as described previously.

### (2) Analysis

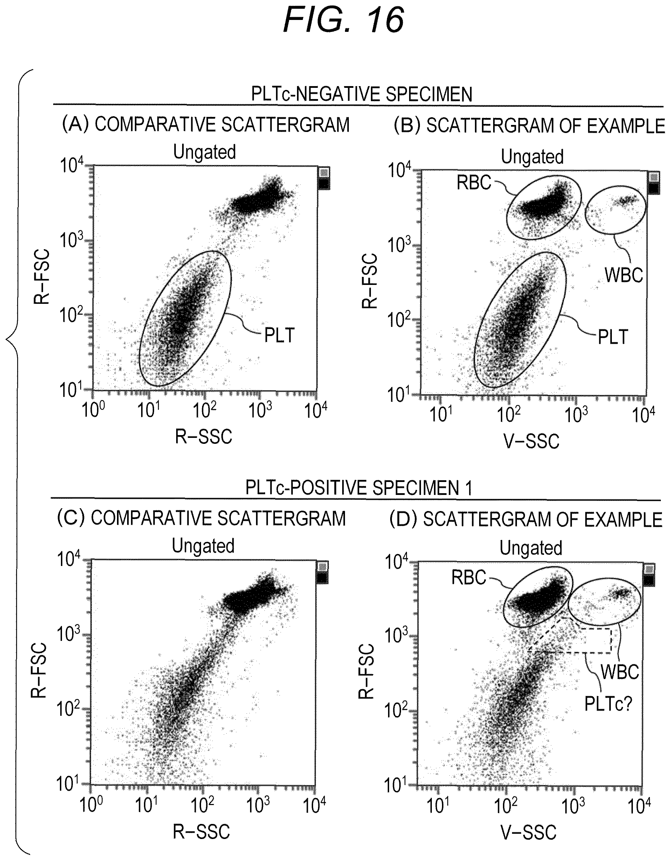

The individual measurement samples were measured with the analyzer, to acquire the first scattered light information, the second scattered light information, and the third scattered light information. The first scattered light information corresponded to the side-scattered light intensity, obtained by irradiating the particles in the measurement sample with a blue-violet laser (also referred to as "side-scattered light intensity (blue-violet)" or "V-SSC", hereinafter). The second scattered light information corresponded to the forward-scattered light intensity, obtained by irradiating the particles in the measurement sample with a red laser (also referred to as "forward-scattered light intensity (red)" or "R-FSC", hereinafter). The third scattered light information corresponded to the side-scattered light intensity obtained by irradiating the particles in the measurement sample with a red laser (also referred to as "forward-scattered light intensity (red)" or "R-SSC", hereinafter). A scattergram was created for each of the measurement samples, with the side-scattered light intensity (blue-violet) plotted on the abscissa, and with the forward-scattered light intensity (red) plotted on the ordinate (also referred to as "scattergram of Example", hereinafter). For comparison, also a scattergram was created for each of the measurement samples, with the side-scattered light intensity (red) plotted on the abscissa, and with the forward-scattered light intensity (red) plotted on the ordinate (also referred to as "comparative scattergram", hereinafter). The individual scattergrams are illustrated in (A) to (D) in Figs. 16 to 18. Hereinafter in the drawings, "WBC" stands for white blood cell, "RBC" stands for red blood cell, "PLT" stands for platelet, and "PLTc" stands for platelet aggregation.

Referring now to the comparative scattergram of the PLTc-negative specimen named (A) in Fig. 16, the platelet population appeared in an area where R-FSC is low. Platelet, having the size smaller than white blood cell and red blood cell, demonstrated lower R-FSC. Hence, the comparative scattergram could identify thereon the platelet population. White blood cell and red blood cell were however appeared in an overlapped manner. The comparative scattergram therefore could not identify white blood cell and red blood cell. On the other hand, referring now to the scattergram of the PLTc-negative specimen in Example, named (B) in Fig. 16, the white blood cell population, the red blood cell population, and the platelet population were clearly classified. Red blood cell demonstrated low V-SSC, supposedly because hemoglobin in red blood cell absorbs the light of the first wavelength (blue-violet), as has been described previously. The red blood cell population on the scattergram named (B) in Fig. 16 was thus considered to appear in an area where V-SSC is lower than that of white blood cell. As can be seen, the scattergram of Example could discriminate white blood cell, red blood cell, and platelet.

Referring now to the comparative scattergram of the PLTc-positive specimen 1 named (C) in Fig. 16, observed was a particle group that distributes to extend from the platelet population towards the population where white blood cell and red blood cell overlap. Whether or not the particle group represents red blood cell or platelet aggregation, however, could not be determined. As can be seen, platelet aggregation was hardly detectable with reference to the side-scattered light intensity and the forward-scattered light intensity obtained only with the red laser. On the other hand, referring now to the scattergram of the PLTc-positive specimen 1 in Example, named (D) in Fig. 16, the white blood cell population, the red blood cell population, and the platelet population were clearly classified. On this scattergram, there appeared a particle group that was not observed on the scattergram named (B) in Fig. 16, in an area surrounded by the broken line. Platelet aggregation is larger in size than normal platelet, and this makes the forward-scattered light intensity higher. The particle group in the area surrounded by the broken line was thus discriminable as the platelet aggregation. On the scattergram named (D) in Fig. 16, a flag "PLTc?" was used to indicate appearance of the particle suspected of forming platelet aggregation. On the scattergrams in Example, appearance of platelet aggregation was clearly recognized, since the red blood cell population appeared in the area where V-SSC is lower than that in the comparative scattergrams. As can be seen, platelet aggregation was proved to be detectable, with reference to the side-scattered light intensity obtained by the blue-violet laser, and the forward-scattered light intensity obtained by the red laser.

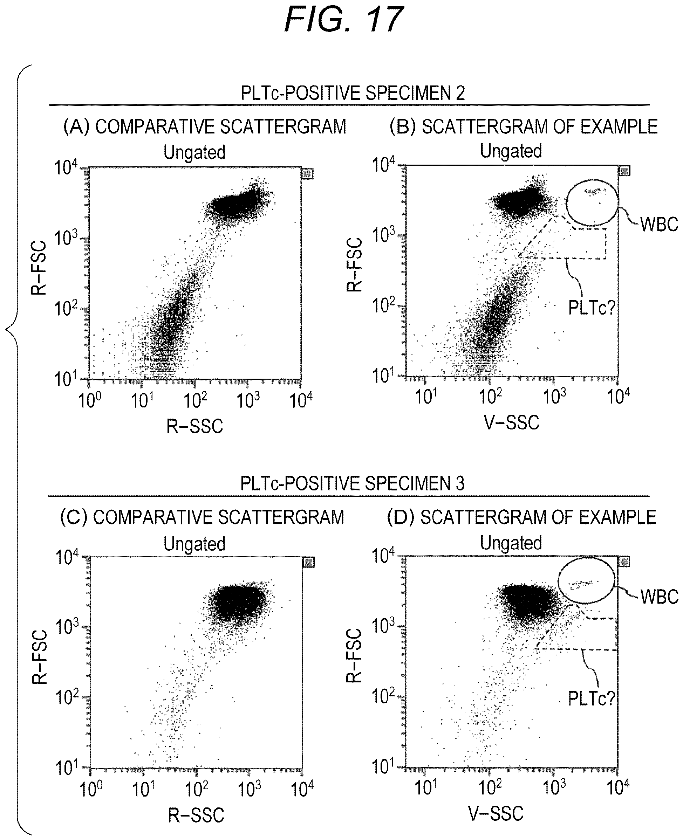

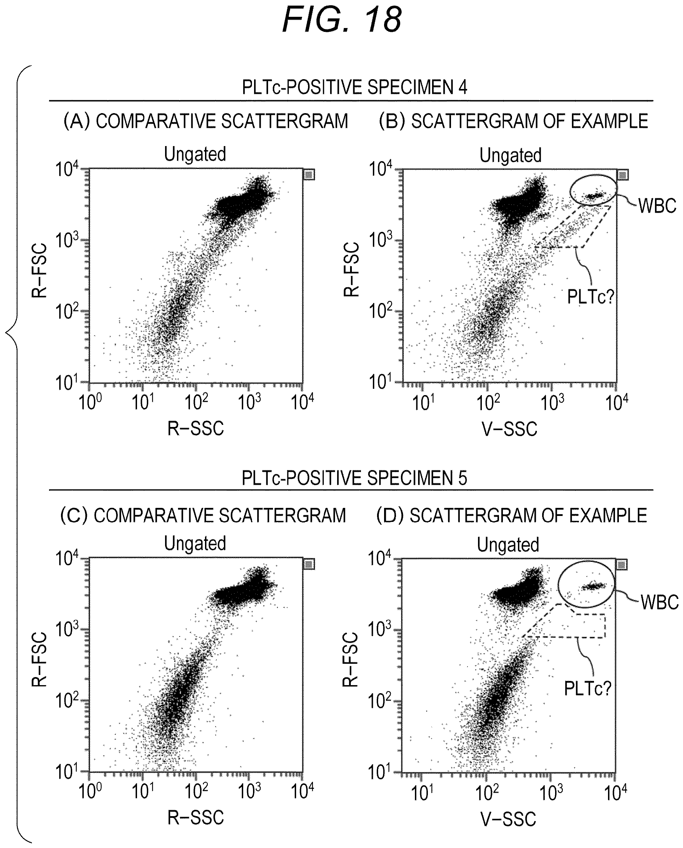

Referring now to the comparative scattergram named (A) in Fig. 17, whether or not platelet aggregation is contained in the PLTc-positive specimen 2 could not be determined. In contrast, referring now to the scattergram of Example named (B) in Fig. 17, particles suspected of forming platelet aggregation were observed in an area surrounded by the broken line. Referring now to (C) in Fig. 17, the PLTc-positive specimen 3 was found to contain less amount of platelet. Whether or not the PLTc-positive specimen 3 contains platelet aggregation was not determined. Referring now to (D) in Fig. 17, particles suspected of forming platelet aggregation were however observed in an area surrounded by the broken line. Referring now to (A) in Fig. 18, the PLTc-positive specimen 4 demonstrated a particle group that is not discriminable between red blood cell and platelet aggregation, like in the case of the PLTc-positive specimen 1. In contrast, referring now to the scattergram of Example named (B) in Fig. 18, particles suspected of forming platelet aggregation were observed in an area surrounded by the broken line. Referring now to (C) in Fig. 18, whether or not platelet aggregation is present could not be determined for the PLTc-positive specimen 5, like in the case of the PLTc-positive specimen 2. In contrast, referring now to the scattergram of Example named (D) in Fig. 18, particles suspected of forming platelet aggregation were observed in an area surrounded by the broken line. As can be seen, the scattergrams of this Example were proved to successfully detect platelet aggregation in various types of blood specimens.

### Example 2: Analysis of Measurement Sample that Contains Platelet Aggregation (2)

Measurement samples were prepared from the blood specimens that contain platelet aggregation, and the platelet aggregation in the measurement samples was detected with an analyzer, in the same manner as in Example 1. For comparison, measurement samples that contain a fluorescent dye were prepared from the same blood specimens, and analyzed in the same manner.

### (1) Measurement Samples

Measurement samples were prepared as described previously, from blood specimens determined to be positive for platelet aggregation by visual observation under a microscope (also referred to as "measurement samples containing a fluorescent dye", hereinafter). Measurement samples containing a fluorescent dye were prepared as follows. First, 2,7,9-trimethylacridine-3,6-diamine as the fluorescent dye was dissolved in a special grade ethylene glycol (1 L) to prepare a staining reagent. Each measurement sample was prepared by mixing 1000 µL of CELLPACK DFL, 5.0 µL of whole blood, and 20 µL of the staining reagent. The staining reagent was in 51.25 × dilution in the measurement sample. The final concentration of the fluorescent dye in the measurement sample was 1.0 ppm.

### (2) Analysis

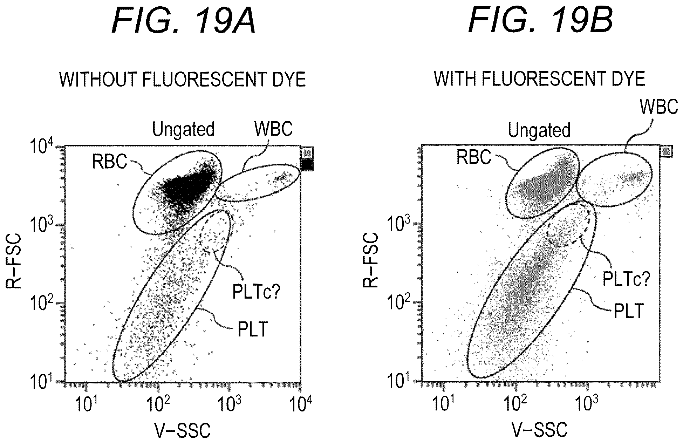

The individual measurement samples were measured with the analyzer, to acquire the first scattered light information and the second scattered light information. The first scattered light information and the second scattered light information were same as those in Example 1. A scattergram was created for each of the measurement samples, with the side-scattered light intensity (blue-violet) plotted on the abscissa, and with the forward-scattered light intensity (red) plotted on the ordinate, in the same manner as in Example 1. The individual scattergrams are illustrated in (A) and (B) in Fig. 19. Referring now to (A) in Fig. 19, the particles in the measurement sample, free of the fluorescent dye, could be classified into the red blood cell population, the white blood cell population, and the platelet population. Also particles suspected of forming platelet aggregation were observed in an area surrounded by the broken line. Referring now to (B) in Fig. 19, also the particles in the measurement sample, containing the fluorescent dye, could be classified into the red blood cell population, the white blood cell population, and the platelet population, like in the scattergram named (A). Also particles suspected of forming platelet aggregation were observed in an area surrounded by the broken line. The method for detecting platelet aggregation of this embodiment, although without use of the fluorescent reagent, was proved to successfully classify red blood cell, white blood cell, and platelet, and to detect platelet aggregation, even if the fluorescent dye is contained in the measurement sample.

## Claims

1. A method for detecting platelet aggregation, the method comprising:
acquiring first scattered light information and second scattered light information generated by irradiating a measurement sample that contains particles with a light of a first wavelength and a light of a second wavelength; and
acquiring information that indicates presence or absence of platelet aggregation, with reference to the first scattered light information and the second scattered light information,
the first wavelength being 315 nm or longer and 600 nm or shorter, and the second wavelength being 610 nm or longer and 750 nm or shorter.

2. The method according to claim 1, wherein the first scattered light information is information related to side-scattered light generated from the particles upon irradiation of the measurement sample with the light of the first wavelength, and
the second scattered light information is information related to forward-scattered light generated from the particles upon irradiation of the measurement sample with the light of the second wavelength.

3. The method according to claim 1 or 2, wherein the measurement sample is prepared by mixing whole blood and a dilution reagent.

4. The method according to any of claims 1 to 3, wherein the measurement sample is free of hemolysis reagent and fluorescent dye.

5. The method according to any of claims 1 to 4, further comprising, before acquiring the first scattered light information and the second scattered light information, preparing the measurement sample by mixing whole blood and a dilution reagent,
the preparing does not include hemolyzing red blood cell contained in the measurement sample, and does not include staining blood cells contained in the measurement sample with a fluorescent dye.

6. The method according to any of claims 2 to 5, wherein the information related to the side-scattered light is side-scattered light intensity, and the information related to the forward-scattered light is forward-scattered light intensity,
in the step of acquiring information that indicates presence or absence of platelet aggregation:
the particles in the measurement sample are classified into a platelet-containing population and a non-platelet blood cell-containing population, with reference to the side-scattered light intensity and the forward-scattered light intensity; and
information that indicates presence of the platelet aggregation is acquired, if the platelet-containing population contains any particle whose forward-scattered light intensity is equal to or above a threshold value.

7. The method according to claim 6, further comprising classifying the non-platelet blood cell-containing population into a red blood cell-containing population and a white blood cell-containing population, with reference to the side-scattered light intensity and/or the forward-scattered light intensity.

8. The method according to claim 6 or 7, wherein in the step of acquiring information that indicates presence or absence of platelet aggregation, a scattergram is created with reference to the side-scattered light intensity and the forward-scattered light intensity, and the platelet-containing population is identified on the scattergram.

9. The method according to any of claims 2 to 5, wherein the information related to the side-scattered light is side-scattered light intensity, and the information related to the forward-scattered light is forward-scattered light intensity,
the acquiring information that indicates presence or absence of platelet aggregation further comprises:
classifying the particles in the measurement sample into a while blood cell-containing population and a non-white blood cell-containing population, with reference to the side-scattered light intensity and the forward-scattered light intensity; and
classifying the non-white blood cell-containing population into a platelet-containing population and a red blood cell-containing population, with reference to the forward-scattered light intensity,
information that indicates presence of the platelet aggregation is acquired, if the platelet-containing population contains any particle whose forward-scattered light intensity is equal to or above a threshold value.

10. The method according to claim 9, wherein in the step of acquiring information that indicates presence or absence of platelet aggregation, a scattergram is created with reference to the side-scattered light intensity and the forward-scattered light intensity, and the platelet-containing population is identified on the scattergram.

11. The method according to any of claims 2 to 5, wherein the information related to the side-scattered light is side-scattered light intensity, and the information related to the forward-scattered light is forward-scattered light intensity, and
in the step of acquiring information that indicates presence or absence of platelet aggregation, a scattergram is created with reference to the forward-scattered light intensity and the side-scattered light intensity, and a particle population that appears in a predetermined area is detected on the scattergram as the platelet aggregation.

12. The method according to claim 11, wherein in the step of acquiring information that indicates presence or absence of platelet aggregation, a scattergram is created with reference to the forward-scattered light intensity and the side-scattered light intensity, and at least one selected from the group consisting of white blood cell, platelet, and red blood cell is classified on the scattergram.

13. The method according to any of claims 1 to 12, wherein the first wavelength is 315 nm or longer and 490 nm or shorter.
